# EUROPEAN PATENT SPECIFICATION

(11) **EP 1 613 230 B1**
(45) Date of publication and mention of the grant of the patent: **03.11.2010**
(21) Application number: 04723454.7
(22) Date of filing: 26.03.2004
(51) Int. Cl.: A61B 18/18, A61N 5/02, A61B 18/14

(54) **A MICROWAVE ANTENNA FOR MEDICAL ABLATION**
MIKROWELLENANTENNE FÜR MEDIZINISCHE ABLATION
ANTENNE HYPERFREQUENCE POUR ABLATION CHIRURGICALE

(30) Priority: 26.03.2003 AU 2003901390
(43) Date of publication of application: 11.01.2006
(73) Proprietor: UNIVERSITY OF TECHNOLOGY, SYDNEY, Broadway, NSW 2007 (AU); Sydney West Area Health Service, Westmead, NSW 2145 (AU)
(72) Inventor: CHIU, Heng-Mao, Sydney, NSW 2000 (AU); SANAGAVARAPU, Ananda, Mohan, Carlingford, NSW 2118 (AU); THOMAS, Stuart, Phillip, Cheltenham, NSW 2119 (AU); ROSS, David, Leslie, Cheltenham, NSW 2119 (AU); GUY, Duncan, James, Ramsay, Glenhaven, NSW 2156 (AU)
(74) Representative: Price, Nigel John King
(86) International application number: PCT/AU2004/000392
(87) International publication number: WO 2004/084748

(56) References cited:
- EP-A- 1 186 274
- WO-A-00/47282
- WO-A-00/49957
- WO-A-98/49933
- WO-A1-00/49957
- US-A- 4 700 716
- US-A- 4 800 899
- US-A- 5 026 959
- US-A- 5 122 137
- US-B1- 6 287 302
- US-B2- 6 527 768

## Description

### Technical Field

This invention concerns a microwave antenna for medical ablation. In particular it concerns such an antenna suitable for cardiac ablation. In a further aspect it concerns a method for making such an antenna.

### Background Art

The heart is composed of three types of cardiac tissue, atrial muscle, ventricular muscle and specialized excitatory and conduction tissues. The atrial and ventricular muscles of the heart are normally excited synchronously. Each cardiac cycle begins with the generation of action potentials by the sino-atrial (SA) node located in the posterior wall of the right atrium. These action potentials spread through the atrial muscle by means of specialized conduction tissue, causing contraction. The action potentials do not normally spread directly from the atrial muscles to the ventricular muscle. Instead, the action potentials conducted in the atrial musculature reach the atrioventricular (AV) node and its associated fibres, which receive and delay the impulses. Potentials from the AV node are conducted to the His-Purkinje (HIS) bundle. This structure carries the impulses to the ventricular musculature to cause their synchronous contraction following contraction of the atrial muscles.

Arrhythmia is a term used to describe irregular beating of the heart. Cardiac arrhythmias generally result from abnormal electrical connections or circuits which form within the chambers of the heart. For example, arrhythmia circuits may form around the veins or arteries.

Episodes of an abnormal increase in heart rate are termed paroxysmal tachycardia. This can result from an irritable focus in the atrium, the AV node, the HIS bundle, or the ventricles. Episodes of tachycardia may be initiated and sustained by either a re-entrant mechanism, or may be caused by repetitive firing of an isolated focus.

Atrial fibrillation occurs in the atrial of the heart, and more specifically at the region where pulmonary veins are located. It is one of the most common arrhythmias with high mortality rate. In the elderly population, those over the age of 80, it has a prevalence of around 10%. One third of all patients who have strokes are in atrial fibrillation when they get to hospital. The atrial fibrillation causes clots in the atria, which travel to the brain to cause the stroke. If a patient goes into atrial fibrillation after a heart attack, the likelihood of fatality doubles. Of all patients with atrial fibrillation the risk of a stroke or similar problem is around 5% per year if not treated.

Each year, around the globe, millions of people are affected by arrhythmias. Many can be treated and are not life-threatening, however, they still claim about 500,000 lives in the United State of America each year.

Current medical treatments rely on pharmaceutical medications which have, at best, a success rate of 50%. Furthermore, these patients may suffer adverse and sometimes life threatening side effects as a result of the medication.

Cutting the arrhythmia circuits is a successful approach to restoring normal heart rhythm. Many different cutting patterns may be implemented to cut arrhythmia circuits. Cardiac ablation involves creating a lesion by use of heat in the myocardial tissue, and has been successfully used to cut arrhythmia circuits. Prior to ablation, the electrical activation sequence of the heart is mapped to locate the arrhythmogenic sites or accessory pathways.

One obsolete ablation approach is the use of high voltage, direct current defibrillator discharges. This approach requires general anaesthesia and can rupture certain cardiac tissues.

Catheter cardiac ablation has recently become an important therapy for the treatment of cardiac arrhythmias, cardiac diarrhythmias and tachycardia. This therapy involves the introduction of a catheter into the veins and manoeuvring it to the reach the heart. An ablation system is then introduced through the catheter to position the ablation source where the tissue is to be ablated.

Radio frequency (RF) catheter ablation systems make use frequencies in the several 100 kHz range as the ablating energy source, and a variety of RF based catheters and power supplies are currently available to the electrophysiologist. However, RF energy has several limitations including the rapid dissipation of energy in surface tissues resulting in shallow lesion, and failure to access deeper arrhythmic tissue. Another limitation is the risk of clots forming on the energy emitting electrodes.

The use of optical and ultrasound energy as ablation sources has also been investigated with limited success. Microwave energy has also been proposed

Document US-A-5 206 959 discloses a device as described in the preamble of claim 1. However, it has proved to be extremely difficult to treat atrial arrhythmias using catheter ablation since it is necessary to produce linear lesions sufficiently long and deep to provide an isolating channel between two conducting nodes. If an effective and continuous linear lesion is not formed the unwanted electrical signal in the heart may be able to find an alternative path. This will cause recurrence of the arrhythmia after the procedure.

### Disclosure of Invention

The invention is as disclosed in the appended set of claims. The invention is a microwave antenna for medical catheter ablation, comprising: A transmission line having an inner conductor, an outer conductor and a dielectric insulator to provide insulation between the inner and outer conductor, An energy emitting antenna element positioned at the distal end of the transmission line to transmit microwave energy. The antenna element has an inner conductor electrically coupled to the inner conductor of the transmission line, and a sheath of dielectric insulator around the inner conductor. A conducting cap is electrically connected to the distal end of the inner conductor, and the cap surrounds a length of the sheath of insulator. The dimensions of the cap are determined to provide impedance matching between the antenna element and the transmission line.

Appropriate impedance matching not only minimises reflections, but also sets up standing waves in the antenna element that assist in producing a near-field having high power.

The particular dimensions of the metallic cap that may be determined, include one or more of:
The length of the cap
The length of the sheath of insulator that is surrounded by the cap
The radius of the cap (the inner radius is determined by the insulator)

The antenna element may be built into the end of the transmission line, and the cap may be soldered to the inner conductor of the transmission line to ensure high physical integrity to the antenna. In particular, a first length of the outer conductor may be removed from the distal end of the transmission line to create the antenna element. A shorter length of the dielectric insulator may be removed from the distal end to expose a length of the inner conductor for fixing of the cap. In this case the dimensions to be determined may further include:
The length of exposed inner conductor between the distal end of the sheath of insulator and the cap.

In one example, the antenna element may be configured with conducting rings, for instance copper or gold, spaced apart from each other along its length by slots. In particular, it may comprise insulating and conducting rings placed alternately along the length of insulating sheath. The insulating rings serve to space the conducting rings apart. An insulating ring is placed first to isolate the adjacent conducting ring from the outer conductor of the transmission line, and last to space the last conducting ring from the cap. In this configuration one or more of the following additional dimensions may be determined:
The width(s) of the conducting rings
The width(a) of the slots (insulating rings)
The length of the antenna element between the end of the outer conductor and the cap.

The conducting rings may comprise rings of outer conductor of the transmission line left in situ.

The cap may be made using a separate conducting ring.

The sizes of the conducting rings and the slots between them affect both amplitude and the phase of the microwave energy being emitted from each slot. As a result they may be selected to determine the shape of the near-field distribution. Making all the conducting rings the same size, and all the slots between them the same size, results in a uniform near field distribution along the length of the antenna element. An optimum configuration may involve the conductive rings being twice as wide as the slots between them.

It is an advantage of this slot configuration is that the length of the antenna element can be lengthened or shortened while maintaining 2 uniform near-field distribution, making it ideal for creating linear lesions for the treatment of atrial fibrillation.

The dielectric loading produced by the size of the insulator surrounded by the cap may be optimized to ensure the near-field terminates at the tip of the antenna rather than at the transmission line/antenna element junction. This prevents heating of the transmission line during the ablation procedure.

By introducing non-uniformity into the slot and ring sizes, the near field can be directed forward or backward. Increasing the slot and ring sizes gradually towards the tip of the antenna makes a forward firing antenna. This can produce spot lesions useful, for instance, for treatment of tachycardia. Decreasing the slot and ring sizes gradually towards the tip of the antenna makes a reverse firing antenna. This can be useful where the tip of the antenna is in a location where no heating is required.

In an alternative example, the antenna element may be configured by being bent to form an open loop oriented such that it extends transverse to the longitudinal axis of the transmission line. This antenna is able to create a circumferential lesion, for instance, around the pulmonary vein. In this configuration one or more of the following additional dimensions may be determined:
The straight length of the sheath of insulator before bending begins.
The radius of bending between the transmission line and the open loop.
The perpendicular distance between the open loop and the beginning of bending.
The radius of the open loop
The length the cap not surrounding the sheath of insulator
The perpendicular distance between the top of the cap and the transmission line.

The shape of this antenna determines the shape of the near-field. The near-field terminates at both the tip of the antenna and the antenna element/transmission line junction. When in situ within a vein, unwanted heating of the transmission line/antenna element junction is reduced by the cooling effect of blood flow.

The antenna may further comprise a Teflon sheath surrounding at least the antenna element. This ensures electrical safety and biocompatibility.

The antenna element may be delivered to an ablation site by feeding the transmission line through a catheter.

The antenna may further comprise a temperature sensor to sense the temperature of the tissue being ablated by the antenna.

The microwave generator may deliver energy at 2.45 GHz or at any other suitable frequency.

A computer control system may be provided to monitor the ablation process and control the microwave generator.

Microwave catheter cardiac ablation offers an alternative treatment modality for patients whose heart rhythm disorders are not responsive to drug therapy, or patients who are too weak for open-heart surgery. It offers the treatment efficacy of open-heart surgery without the associated trauma and post-operative intensive care.

The microwave antenna may be used for ablation, for hyperthermia and for coagulation treatments,

One of the many advantages of tissue ablation using energy at microwave frequencies is that the microwave energy can be delivered to the myocardium without physical contact between the antenna and the myocardium.

In a further aspect the invention is a method for making a microwave antenna for medical catheter ablation, comprising an energy emitting antenna element having an inner conductor and a surrounding sheath of insulation, in use, located at the end of a transmission line. The method comprises the steps of:
forming a conductive cap at the distal end of the antenna element such that it surrounds a length of the sheath of insulator;
electrically coupling the conducting cap to the inner conductor of the antenna element; and
determining the dimensions of the cap to provide impedance matching between the antenna element and the transmission line.

### Brief Description of the Drawings

Two examples of the invention will now be described with reference to the accompanying drawings, in which:
Figure 1(a) is longitudinal section through a coaxial ring slot array antenna.
Figure 1(b) is an end view of the antenna of Figure 1(a).
Figure 1(c) is an exploded diagram of the antenna of Figure 1(a).
Figure 2(a) is a graph showing the normalized near-field across each corresponding slot of the antenna of Figure 1.
Figure 2(b) is a graph showing the reflection coefficients of the antenna of Figure 1.
Figure 2(c) is a graph showing the normalized SAR level for the antenna of Figure 1.
Figure 2(d) is a plot showing the E-field vector of the antenna of Figure 1.
Figure 2(e) is a graph of the measured and simulated reflection coefficients of the antenna of Figure 1.
Figure 2(f) is a graph showing the measured and simulated input impedance of the antenna of Figure 1.
Figure 2(g) is a graph showing the measured temperature distribution of the antenna of Figure 1.
Figure 2(h) is a graph showing the measured temperature at various depths using various power settings.
Figure 2(i) is a graph showing maximum temperature obtained at various depths for different power level.
Figure 3(a) is a pictorial diagram of a parallel loop antenna.
Figure 3(b) is a top elevation view of the antenna of Figure 3(a).
Figure 3(c) is an end elevation view of the antenna of Figure 3(a).
Figure 3(d) is a right elevation view of the antenna of Figure 3(a).
Figure 4(a) is a plot of the E-fiold vector in the X-Y plane for the antenna of Figure 3.
Figure 4(b) is a plot of the E-field vector in the Y-Z plane for the antenna of Figure 3.
Figure 4(c) is a graph of the effect of loop radius, on the reflection Coefficients of the antenna of Figure 3.
Figure 5 is a flow chart illustrating the stops involved in creating lesion.
Figure 6 is a block diagram showing ablation modalities.
Figure 7 is a flow chart for the Fixed-Power fixed-Time (FPFT) ablation modality.
Figure 8 is a flow chart for the temperature modulated microwave ablation modality.
Figure 9(a), (b) and (c) are graphs of the power delivery waveforms for three different ablation modalities.
Figure 10 is a graph of temperature and power waveform for the C/V duty cycle pulsed ablation modality.
Figure 11 is a graph of temperature and power waveform for the Temperature Modulated pulsed ablation modality.

### Best Mode for Carrying Out the Invention

### The Coaxial Ring Slot Array (CRSA) Antenna

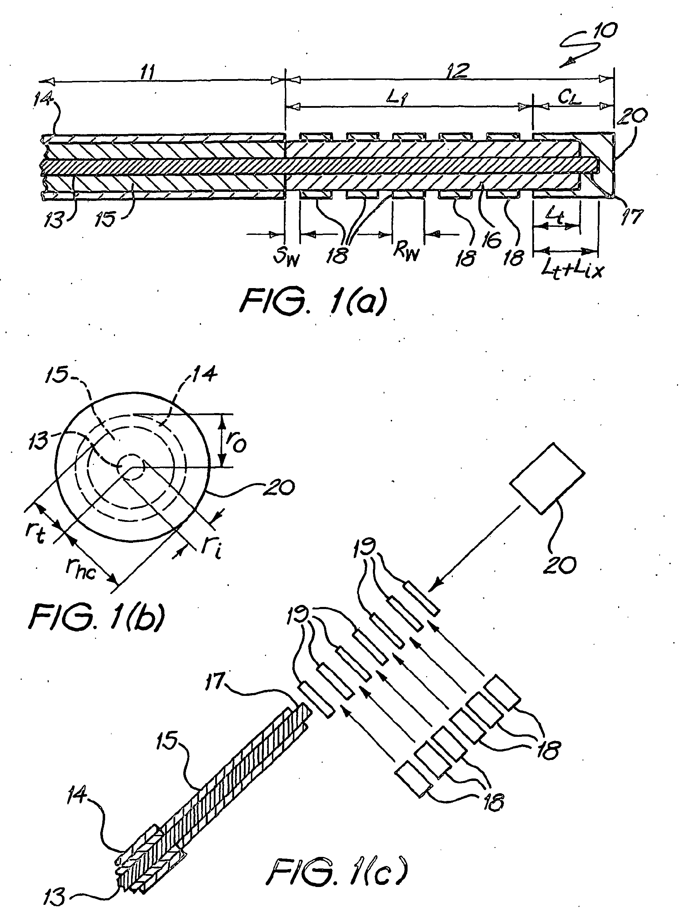

Figure 1 shows the configuration of the coaxial ring slot array (CRSA) antenna 10 for cardiac ablation. The antenna 10 has a coaxial cable transmission line 11 and an antenna element 12 formed at the distal end of the transmission line 11. The coaxial cable comprises an inner conductor 13, an outer conductor 14 and a Teflon dielectric insulator 15 which provides insulation between the inner 13 and the outer 14 conductors. The insulator 15 has a diameter of about 3 mm, and the conductors about 0.91 mm.

The antenna element 12 is constructed out of the distal end of the transmission line by first removing the outer conductor 14 of the coaxial cable for the length L*₁*+L*ₜ*+L*ᵢₓ*, exposing a sheath 16 of insulator 15. At the distal end of the antenna element 12 a short length L*ᵢₓ* of the Teflon insulation sheath 16 is removed exposing an equally short length 17 of the inner conductor 13. Copper rings 18 are made from copper tubes with diameter r*_{Rc}*, and the rings have a width of R*_{w}*. Similar dielectric spacer rings 19 are made out of Teflon material of width S_{w}. The dielectric spacer rings 19 are shown only in Figure 1(c) for the sake of simplicity.

A first dielectric spacer 19 is slid onto the exposed insulator followed by the first copper ring 18. This electrically isolates the capper ring 18 from the outer conductor 14. The procedure is then repeated until all the dielectric spacers 19 and copper rings 18 are in place, and all the copper rings 18 are isolated from each other.

When the last, insulating, ring has been slipped onto the distal end of the antenna element, the Teflon sheath tends beyond the rings for a short distance Lᵣ a 'perturbation distance'. To seal off the distal end hollow copper cap 20 is located partly surrounding the Teflon sheath, but with its distal end extending beyond the end of the Teflon sheath. Then, both the distal end of the cap 20 and the exposed length 17 of inner conductor 13 are pro-heated with soldering gun, and solder is then melted within the hollowed section between the ring and the inner conductor. When the solder cools, it fuses the inner conductor together with the cap and the cap is partially tilled with dielectric insulator. The cap 20 is integrated onto the end of the inner conductor, Between the end of the outer conductor 14 and the cap 20 there is an antenna element 12 comprising copper rings separated by radiation emitting slots.

The cap 20 may be made using one of the copper rings

The antenna is constructed using TFLEX-402 flexible coaxial cable. It can be seen in Figure 1(a) that the radius of the rings 18 and 19 is the same as the radius of the outer conductor of the coaxial cable, The radius the of the cap 20 is also seen to be the same as the outer conductor in this Figure. This allows easy insertion of the antenna into the heart via catheters. Figure 1(b) shows that the cap radius may be larger than the cable, as this is a variable dimension.

A Teflon sheath (not shown) encapsulates the entire antenna 12 to finish construction.

Figures 1(a) and 1(b) show the constitutive parameters for this antenna:
Static Dimensions
   ➢ η : Radius of the inner conductor of the coaxial cable,
   ➢ rᵢ : Radius of the PTPE dielectric,
   ➢ rₒ : Radius of the outer conductor of the coaxial cable,
Variable Dimensions
   ➢ r_{hc} : Radius of the cap,
   ➢ S_{wi} : Width of the slots (insulating rings) between the copper rings,

   - R*_{w}*: Width of the copper rings,
   - L*ᵢₓ*: Length of the exposed inner conductor between the distal end of the sheath of insulator and the cap,
   - L*ₜ*: Length or extension of the sheath of insulator that is surrounded by the cap,
   - L*₁*: Length of the antenna element between the outer conductor and the cap,
   - C*_{L}*: Length of the cap.

For any given length on antenna element (determined by the length of the lesion required), another variable parameter is the number of rings, *N,* which makes *N*+*1* slots in total. The number of lings and slots must be selected in order to achieve a uniform near field distribution. The dimensions of the cap are determined for each length and ring and slot combination.

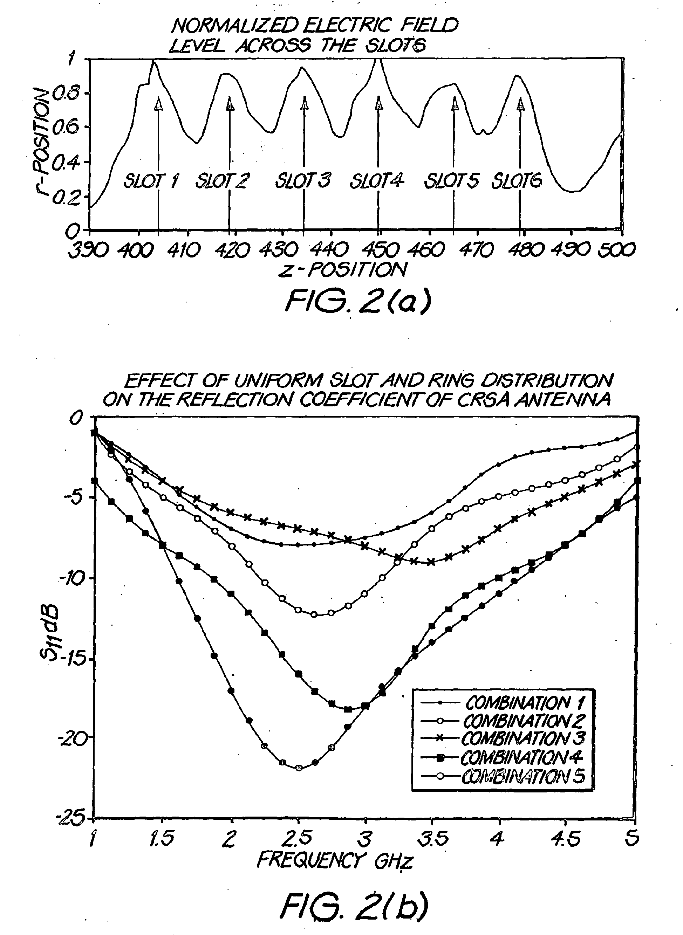

Figure 2(a) shows excitation from multiple sources has been achieved. The normalized near-field distribution across each slot in the antenna also shows only small variation in the magnitude of the near-feld distribution along the length of the antenna element.

The spacing between the slots determines the phase of the microwave radiation, and therefore its primary direction. Uniform near-field amplitude and phase distributions results in coherent radiation emission in the near-field, thus forming a lesion of linear shape.

### Iterative Procedure

An iterative procedure is used to determine the dimensions of the constitutive parameters of the CRSA antenna. Firstly, the iterative procedure is used to obtain the optimum dimensions for the slots and rings for the CRSA antenna. The antenna is to be used for creating linear lesions, the length of the CRSA antenna is adjusted to suit the length of the lesion; in this example the length of the antenna is selected to be 20mm.

Figure 2(b) shows the effect changes of the slot and ring sizes have on the reflection coefficients of the CRSA antenna. Five different combinations have been used in order to obtain the optimum slot and ring sizes in order to achieve lowest antenna return loss. The combinations are shown in Table 1.

**Table 1: Slot and ring combinations used to obtain the reflection coefficient of CRSA antenna shown in Figure 2(b)**

| | Ring Size (nm) | No. Rings | Slot Size (mm) | No. Slots |
|---|---|---|---|---|
| Combination 1 | 10 | 1 | 4 | 2 |
| Combination 2 | 4 | 2 | 4 | 3 |
| Combination 3 | 1 | 9 | 2 | 10 |
| Combination 4 | 1 | 10 | 1 | 11 |
| Combination 5 | 2 | 5 | 1 | 6 |

From Figure 2(b) it can be seen that a CRSA antenna with two large slots and one large ring (combination 1) is not an efficient radiator as indicated by its high energy reflection at 2.45 GHz. As the number of rings and slots increases, it is possible to lower the reflections of the CRSA antenna to a minimum at 2.45 GHz. By using an iterative procedure, the optimum widths for the slots and rings are found to be 2mm for the rings and 1mm for the slots. This is shown in Figure 2(b) as combination 5 which clearly gives the lowest reflection at 2.45 GHz. It should be pointed out here that the cap dimensions are adjusted, after the dimensions of the slots and rings have been determined, to take account of the final dimensions of the slots and rings. The dimensions of the cap for each of the five slot-ring combinations are shown in Table 2.

**Table 2: Cap dimensions (in mm)**

| | Cap Length (C_{L}) | Dielectric Length in the Cap (Lₜ) | Exposed Inner Conductor Length in the Cap (L*ᵢₓ*) | L*ᵢ* + L*ᵢₓ* |
|---|---|---|---|---|
| Combination 1 | 4 | 2 | 1 | 3 |
| Combination 2 | 4 | 1 | 2 | 3 |
| Combination 3 | 4 | 1 | 3 | 4 |
| Combination 4 | 3 | 1 | 1 | 2 |
| Combination 5 | 4 | 2 | 1 | 3 |

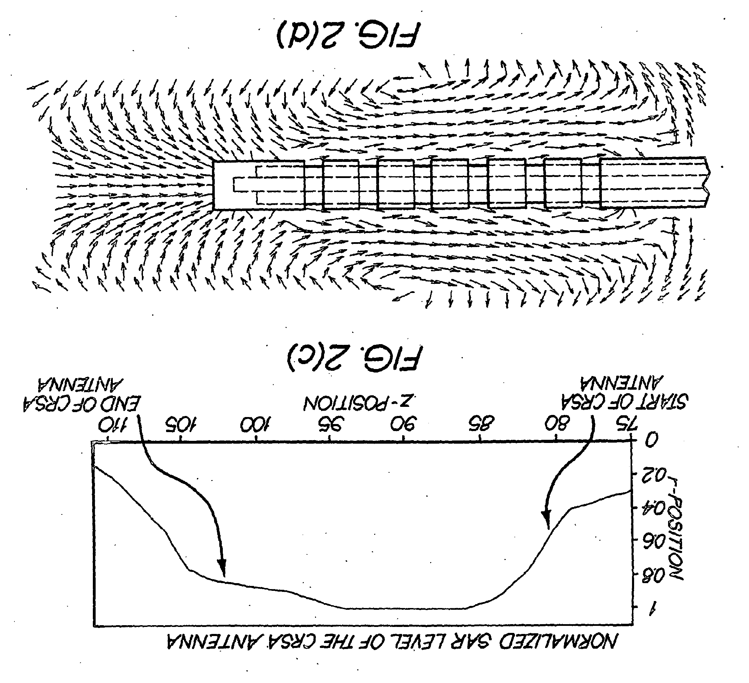

Figure 2(c) shows the normalized specific absorption rate (SAR) level of the CRSA antenna of Figure 2 (d). It can be seen that the SAR level remains almost substantially flat across the entire length of the CRSA antenna.

This flat characteristic is also seen when the flow of the near-field, that is the E-field vector, is plotted, see Figure 2(d). It can be seal that the flow of E-fleld across each of the slots is very smooth thereby generating uniformly distributed SAR. It should also be pointed out that by optimizing the size of dielectric loading inside the cap at the end of the CRSA antenna it is possible to make the E-field terminate at the tip of the antenna rather than to the coaxial cable/antenna junction. This ensures that the coaxial cable will not be unnecessarily heated during the ablation procedure.

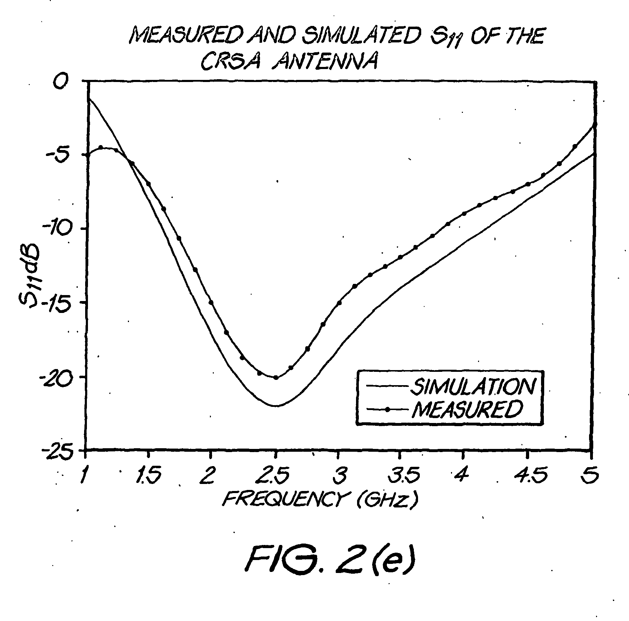

Figure 2(e) shows the simulated and measured reflection coefficients of the CRSA antenna. It is clear that, with optimized slot, ring and dielectric cap dimensions, at the operating frequency of 2.45 GHz the CRSA antenna give very low reflections. This indicates that the CRSA antenna can couple microwave energy efficiently and effectively into the myocardium. Another characteristic of the CRSA antenna shown in this Figure is that it exhibits wide 3dB impedance bandwidth. This is important where the dielectric properties of the surrounding tissue may change with temperature, causing changes in antenna performce. By ensuring that the CRSA antenna has wide 3dB impedance bandwidth, these changes will not decrease the efficacy of the CRSA antenna.

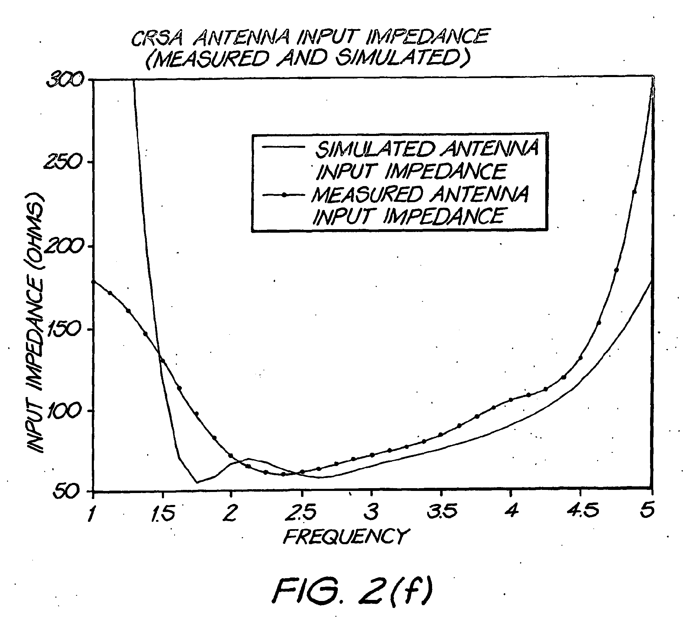

Figure 2(f) shows the simulated and measured input impedance of the CRSA antenna across frequency span of 1 to 5 GHz. As can be seen, the input impedance of the CRSA antenna at 2.45 GHz is very close to the source impedance of 50 Ω. Since the optimized CRSA antenna matches the input impedance of the microwave generator, it is capable of delivering microwave energy without much reflection. The final optimized dimensions for the CRSA antenna are listed in Table 3.

**Table 3: Final CRSA antenna parameter dimensions in mm.**

| | | | | | | | | | | |
|---|---|---|---|---|---|---|---|---|---|---|
| Parameter | r*ᵢ* | r*ₜ* | r*ₒ* | r*_{hc}* | S*_{w}* | R*_{w}* | L*ᵢₓ* | L*₁* | L*_{T}* | C*_{L}* |
| Dimension | 0.255 | 0.816 | 1.071 | 1.1 | 1 | 2 | 1 | 20 | 2 | 3 |

### Thermal Analysis of the CRSA Antenna

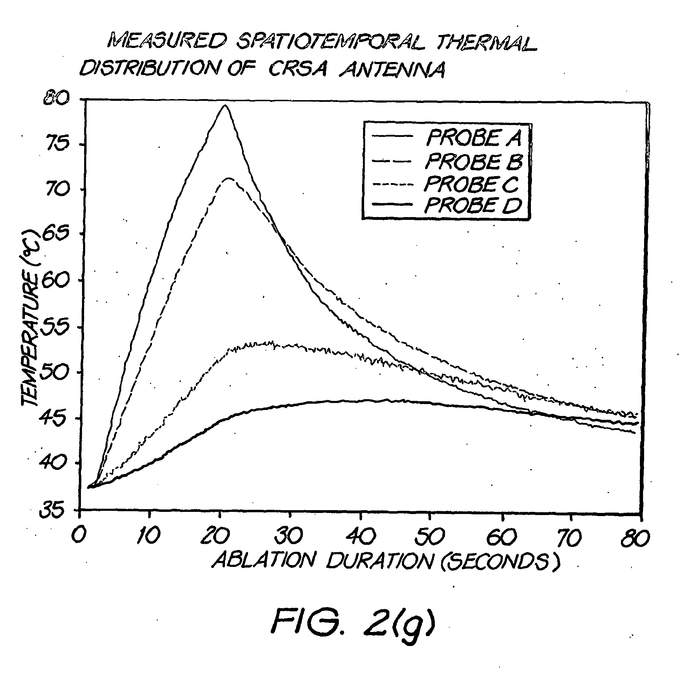

Figure 2(g) shows the spatiotemporal thermal distribution of the CRSA antenna with input power of 80 watts. It can be seen that after 20 seconds of applying microwave energy, the temperature reached by probe A is 80 degrees. This indicates that CRSA is capable of depositing microwave energy effectively into the myocardium thereby reducing the duration of ablation.

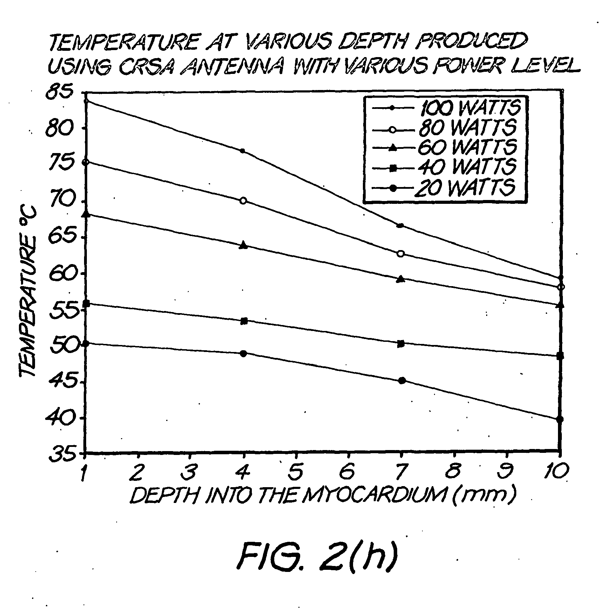

Figure 2(h) shows the plot of measured temperature at various depths into the myocardial tissue using different power settings while the duration is 30 seconds. It can be seen that with an applied power of 100 watts (the diamond line), temperature reached close to 85°C at the surface of the tissue. The temperature drops gradually as the depth of the tissue is increased. At 10 mm deep into the myocardium, the temperature obtainable using 100, 80, 60, 40 and 20 watts are 59°C, 57°C, 55°C, 48°C. and 39°C respectively. From this power-temperature profile, it can be seen that the input power of 60 watts is adequate for the CRSA antenna to achieve irreversible transmural lesions. Also, the radiation will not unnecessarily heat surrounding tissues.

From the same Figure 2(h), it is clear that there is a difference between the temperatures obtainable in the tissue as the power is increased from 40 to 60 watts. Due to the excellent impedance matching, a 50% increase in applied power translates to almost 15°C increase in temperature at the myocardial tissue surface, and 8 °C increase at 10mm deep into the tissue.

This type of thermal characteristic of an antenna is desirable because it means that the CRSA antenna is suitable in a wide range of thermal therapy applications. Since the thermal profiles of applied power of 60 watts or greater are above 55°C, high power can be applied to the CRSA antenna to create necrotic tissues while lower power settings (40 watts or lower) can be applied to the CRSA antenna for hyperthermia applications.

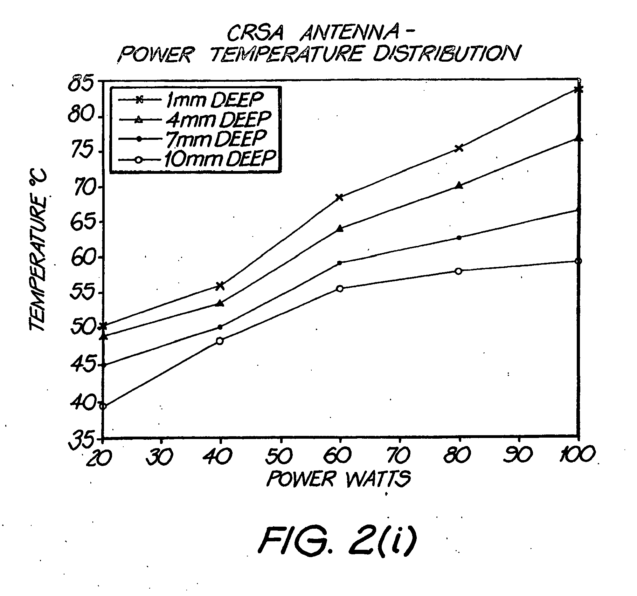

Finally, Figure 2(i) shows the temperature recorded at 1, 4, 7 and 10 mm into the myocardial tissue for various power settings. Again, with 60 watts, the temperature recorded is already exceeding 55°C which indicates 60 watts is the optimum power setting for CRSA antenna for tissue necrosis.

### Field Variation

The ability to generate uniform excitation across uniformly spaced slots and metallic rings is exploited further to define the shape the near-field distribution produced by the CRSA antenna. By introducing non-uniformity into conducting and insulating ring sizes the E-field level towards the tip of the CRSA antenna can be enhanced while minimizing the E-field level near the antenna/cable junction by using narrow slot and ring sizes there. The slot and ring sizes are increased gradually as it approaches the tip of the CRSA antenna, This, in-effect, makes the CRSA antenna as forward firing antenna.

The opposite effect on the neat-field distribution can also be achieved by reversing the slot and ring sizes on the CRSA antenna. That is, the width of the slots and rings near the antenna/cable junction is made larger and their sizes gradually decrease as they approach the tip of the antenna. This makes a reverse firing antenna.

The possibility of adjusting the near-field distribution by changing the slot-ring compositions makes the CRSA antenna useful for various types of ablation. The forward firing antenna is useful for creating short linear lesions as well as spot lesions used in the treatment of ventricular tachycardia. The reverse firing antenna is useful for producing lesions near the antenna/cable junction but not towards the tip of the antenna, this can be useful for instance where the tip of the antenna is extended to areas where heating should be minimized. An example of this is where the tip of the antenna may be extended over the tricuspid valve during the ablation of the atriventricular node for the treatment of atrial fibrillation.

### The Parallel Loop (PL) Antenna

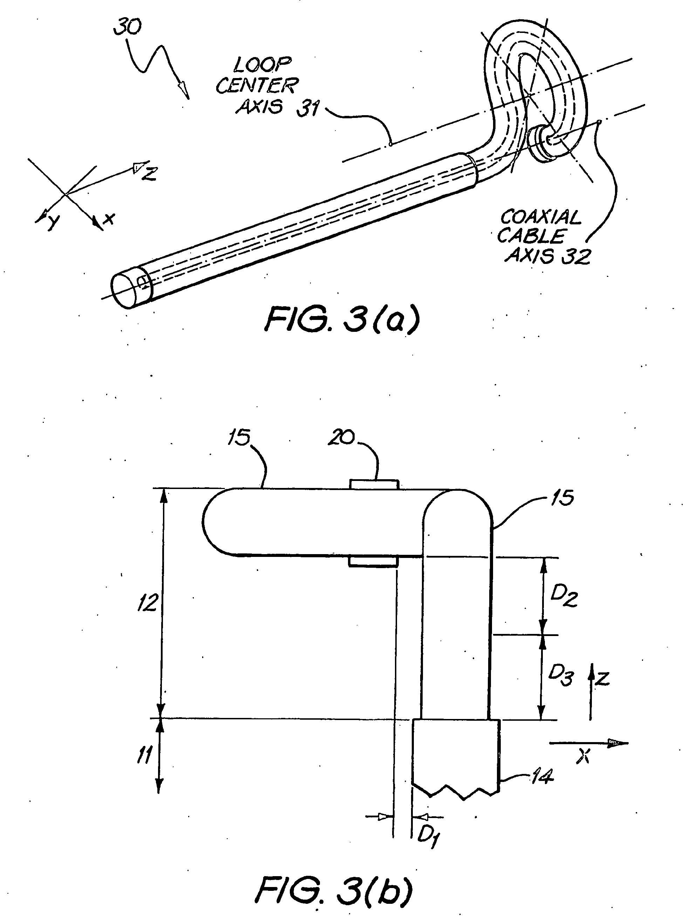

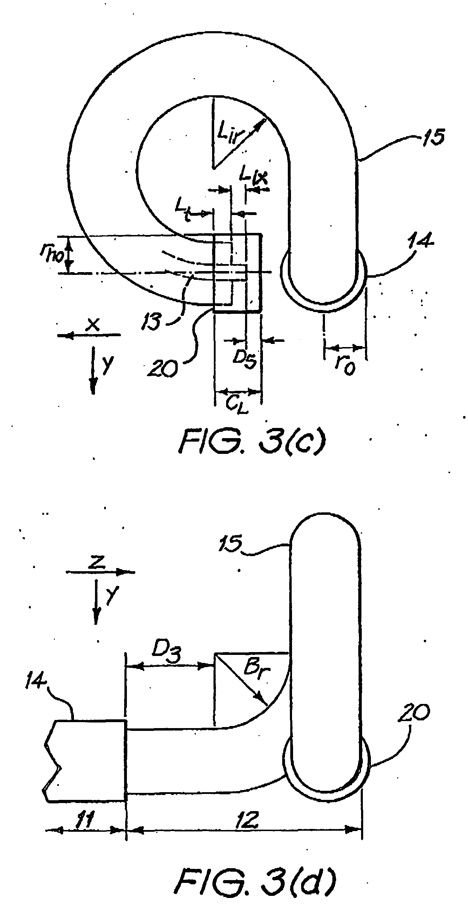

The configuration of the parallel loop (PL) antenna 30 is shown in Figures 3(a) to 3(d), the same reference numerals have been used as in Figure 1 to identify the corresponding features. This antenna is used to create circumferential lesions around the pulmonary vein. It is called the parallel loop antenna because the center axis 31 of the loop antenna section is parallel to the axis 32 of the coaxial cable.

The constitutive parameters of a PL antenna are:
Static Dimensions
   - r*ᵢ*: Radius of the inner conductor of the coaxial cable,
   - r*ₜ*: Radius of the PTFE dielectric,
   - r*ₒ*: Radius of the outer conductor of the coaxial cable,
Variable Dimensions
   - r*_{hc}*: Radius of the cap.
➢ D₁: The perpendicular distance between the top of the cap and the transmission line.
➢ D₂: The perpendicular distance between the open loop and the beginning of bending.
➢ D₃: The straight length of the sheath of insulator before bending begins.
➢ Lᵢₓ: Length of the exposed Inner conductor between the distal end of the
➢ Lₜ: Length of the sheath of insulator that is surrounded by the cap.
➢ D₅: The length the cap not surrounding the sheath of insulator (D₅ = C_{L} - (L_{I} + Lᵢₓ)
➢ L₁: Length of the antenna element between the outer conductor and the cap,
➢ C₁: Length of the cap.
➢ Lᵢᵣ: The radius of the open loop.
➢ Bᵣ: The radius of bending between the transmission line and the open loop.

The variable parameters are determined using an iterative procedure as before.

The loop antenna is designed to create a near-field that surrounds the entire loop element, but extends very little along the coaxial cable section. This feature enables the loop antenna to create a circumferential lesion along the wall of the pulmonary vein.

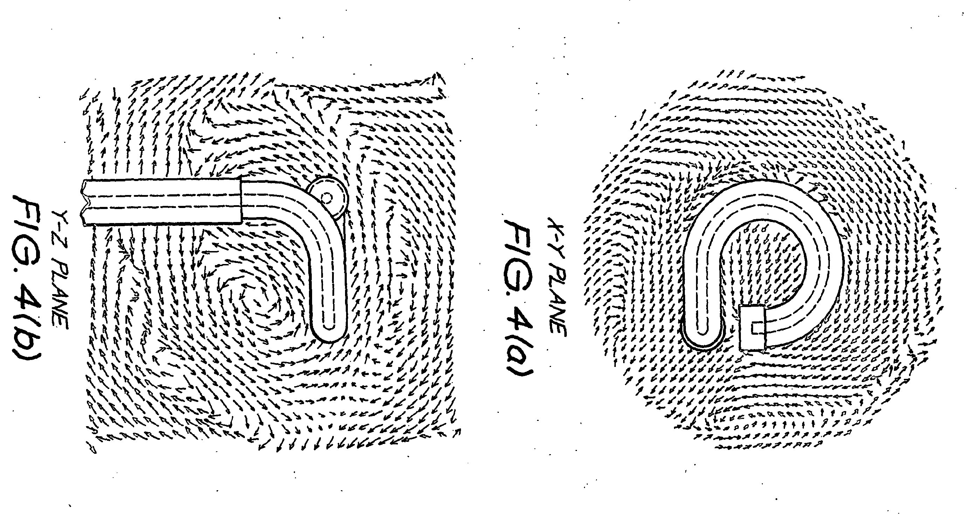

Evidence of the confinement of the near-field to the loop element section of the PL antenna is shown on the vector plot of the B-field flow, see Figure 4(a) and (b). On the X-Y plane it can be seen that the B-field emitted from the loop section of the PL antenna uses the cap as the return path.

Apart from the loop antenna feeding section, most of the areas surrounding the loop antenna are being exposed to same level of SAR. This is made possible due tₒ the fact that the areas surrounding the loop antenna section are very evenly illuminated by E-fields.

The areas immediate surrounding the PL antenna has very high level of SA1 where most of beating occurs. The SAR value decreases rapidly as the distance away from the loop section of the PL antenna increases and that by the time the E-field reaches the point furthest away from the PL antenna the BAR value has already dropped to less than 50% which is not sufficient to create any irreversible damage to the tissue under the short time duration used for ablation. This is a desirable safety feature of the PL antenna as the areas immediate surrounding the pulmonary vein are heated, the tissue outside of the pulmonary vein, although will also be heated, but irreversible damage on the tissues outside of the pulmonary vein will not be made.

On the other hand, the near-field emitted from the bending section of the loop antenna uses the coaxial cable/antenna junction as the return path as intended. Due to the complexities of the PL antenna, two return paths are required in order to confine the near-field to the loop section of the PL antenna. Although the use of cable/antenna junction as the return path for parts of the near-fields can cause hot spots to form around the cable/antenna junction area, the rate of blood flow in the pulmonary vein is high enough to provide adequate cooling to the PL antenna.

### Iterative Procedure

In order to produce such results an iterative procedure is used to optimize the PL antenna.

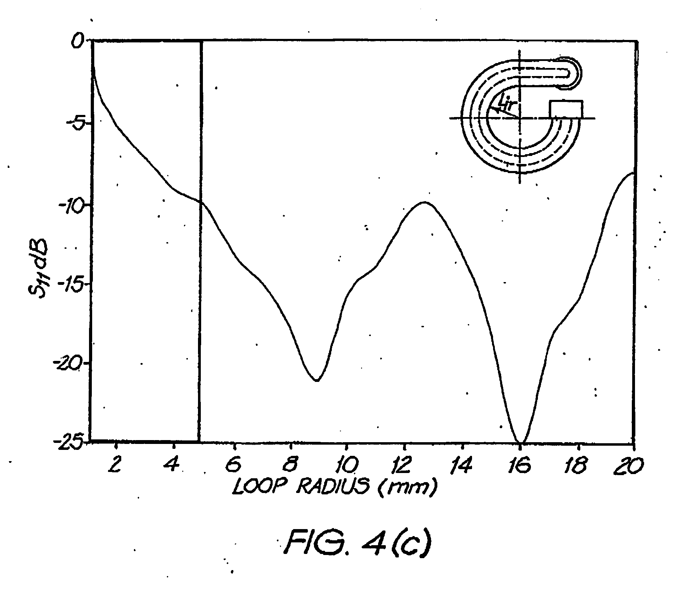

There are many variable constitutive parameters of the PL antenna, and all can be optimized. In order to accelerate the optimization procedure, the iterative optimization of the PL antenna is separated into two sections:
Firstly, the radius of the open loop L*ᵢᵣ* is optimized. The reflection coefficient of various loop sizes are obtained, see Figure 4(c) which plots the reflection coefficient of the PL antenna based on the radius of the open loop. From Figure 4(c) it is clear that the size of the loop has strong effect on the reflection coefficient of the loop antenna. There are two local minima, but since 16mm diameter is too big for insertion into the pulmonary vein, L*ᵢᵣ* is selected to be 9 mm.

Once the optimum dimensions for the loop have been obtained, the iterative procedure then proceeds to optimize the bending radius B*ᵣ* based on the dimensions obtained for the loop section. The cap of the PL antenna is then optimized. Other dimensions such as the amount of exposed insulation before bending D*₃*, and the amount of insulation and inner conductor in the cap have a direct affect on the return loss of the antenna, and are also optimized. The distance away from the cable/antenna junction before the bending of the loop section of PL antenna starts, is bounded by construction constraints to be no less than 3 mm and no more than 10mm.

### Microwave Ablation System Hardware And Software Development

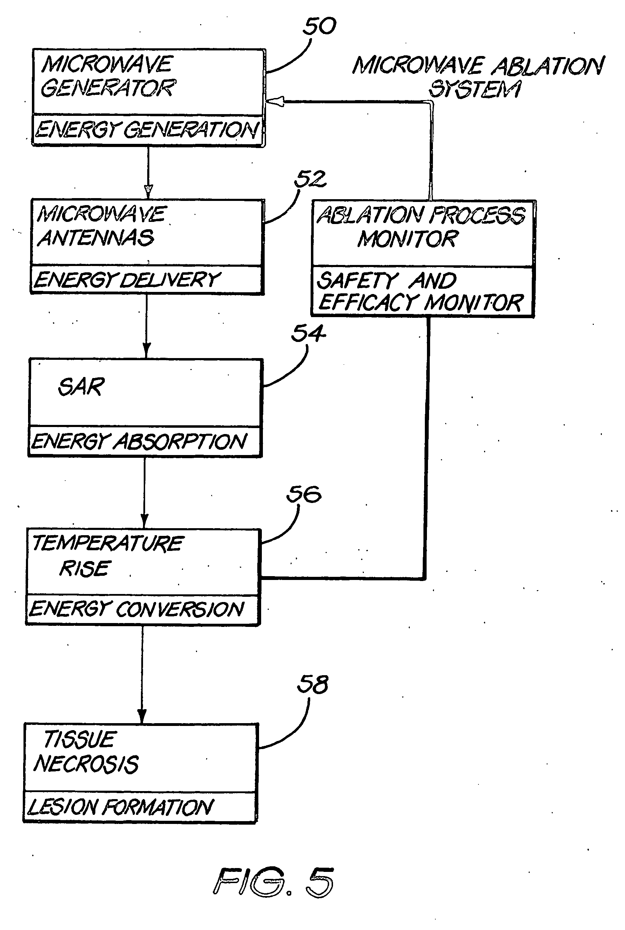

Figure 5 shows the process for creating lesions. First of all, microwave energy is generated 50 by a microwave generator. The energy is then delivered 52 to the myocardial tissue by microwave antennas 12 and coaxial cable. Fart of the delivered energy is absorbed 54 by the myocardial tissue and part of the delivered energy is reflected or lost in the surrounding materials. The energy absorbed by the tissue then causes the tissue temperature to rise 56 to a point where tissue necrosis occurs 58 and subsequently the lesions are formed.

### Interface Card

An interface card links the remote control interface, see Table 4, of the microwave generator to a notebook computer to automate monitoring of forward/reflected power, ablation duration and switching the generator on and off. A serial interface is used to connect the microwave generator to a notebook computer to be remote controlled.

**Table 4 Remote control interface pin assignment.**

| Pin Number | Description |
|---|---|
| 1 | Remote external interlock. (High = Remote Control) |
| 2 | External interlock chain status. (High = On) |
| 3 | Operation Status. (High = In operation) |
| 4 | Operation/Standby status. (Maintained High = Operation) |
| 5 | Reflected power monitor. (0 to 5 volts = 0 to 250 watts) |
| 6 | Forward power set. (0 to 5 volts = 0 to 250 watts) |
| 7 | Forward power monitor. (0 to 5 volts = 0 to 250 watts) |
| 8 | Remote reflected power input. |
| 9 | Remote external interlock. |
| 10 | Circuit ground. |
| 11 | Circuit ground. |
| 12 | Circuit ground. |
| 13 | Circuit ground. |
| 14 | Circuit ground. |
| 15 | + 15V DC* |

In order to protect the notebook computer from power surges which may be caused by the reflected power from the load, the notebook computer is isolated from the microwave generator using opto-isolator transistors on the digital data line and the - two analog forward and reflected power monitoring lines.

In order to provide enough current to drive the opto-isolators as well as the multiplexer switch in the interface card, +15 volt DC is tapped from a pin of the microwave generator. A voltage regulator is used to provide a regulated +5 volt DC to supply to the opto-isolators and the multiplexer switch.

The interface card also provides a RS-232 terminal to connect to the temperature measurement system for the recording and monitoring of temperatures during ablation.

A graphical user interface is provided for the notebook computer.

### Control and Monitoring Software

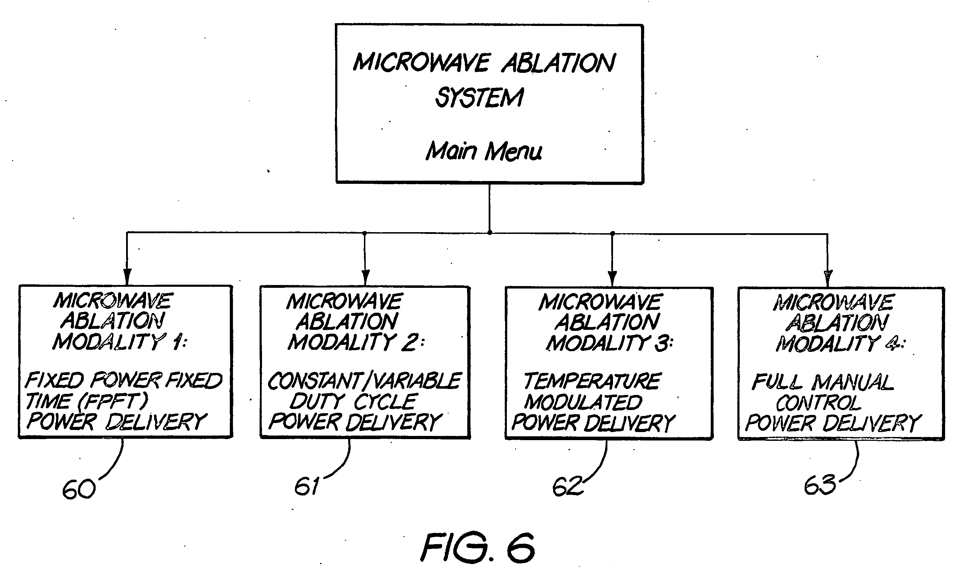

Figure 6 shows the structure of the microwave ablation control and monitor software. The way microwave energy is delivered to the myocardium is dependent on which ablation modality the cardiologist chooses.

The first ablation modality 60 requires the microwave generator to output a predefined level of energy for a predefined time.

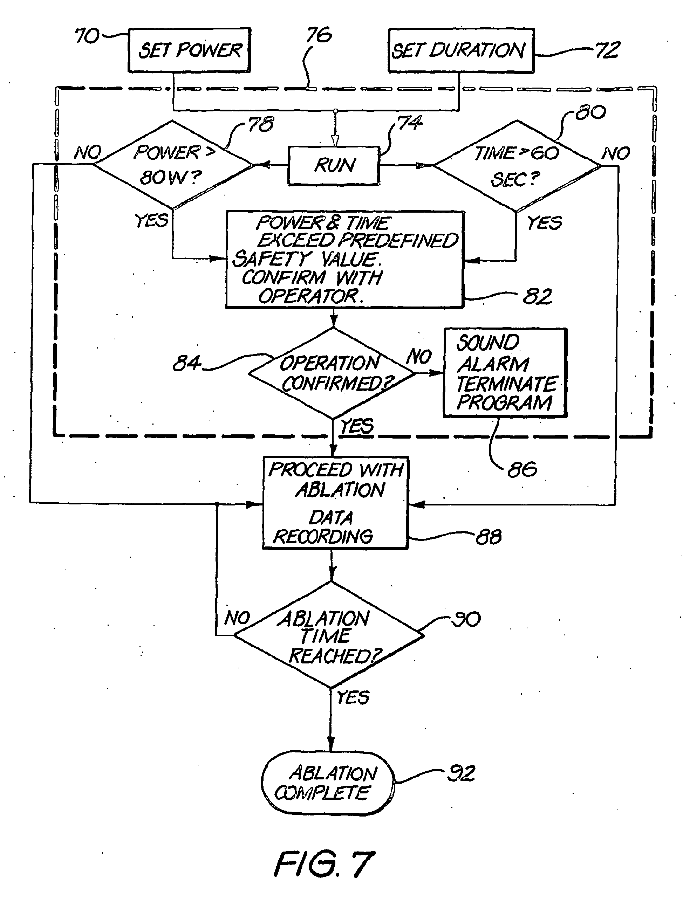

Referring to Figure 7, the power output is set. An analog meter displays the actual power being delivered. To increase the flexibility of the power delivery mode, the power can be increased or decreased in real time. Even though the microwave generator is capable of generating 250 watts, the maximum power output is electrically limited to 100 watts for safety reasons.

The ablation duration is then set 72, in seconds. Similar to the power setting, the total ablation duration can be increased or decreased in real time during ablation depending on the cardiologist's decision.

Once the power and time arc defined, the Run/Stop toggle switch can be pressed 74 to initiate the pre-ablation checking sequence 76. The pre-ablation check sequence consists of checking if the power setting is higher than 80 watts 78 and that if the time is longer than 60 seconds 80. If this combination is detected, then an alarm will sound and the cardiologist is required to confirm the entered power/time combination 82. If the cardiologist confirms 84 that the power/time settings are correct, then the program proceeds and the ablation procedure is initiated. A real time display is provided of the tissue temperature during ablation. However, on the other hand, if no confirmation is received the program is terminated 86.

Once the program enters the ablation stage 88, the program continues to execute until the preset ablation duration is reached 90 and then the program, hence the ablation procedure, is terminated 92. It should be pointed out that the Run/Stop switch also acts as an emergency stop switch which is electronically-wired to the space bar.

Once the ablation procedure is terminated, the recorded temperature, together with power and time settings, are saved in the local hard disk of the notebook computer for record keeping and further analysis if necessary.

During ablation, the recorded temperature is used to monitor the tissue temperature. If the temperature is over a preset level, even if the ablation time is not reached, the program will terminate hence stopping the ablation procedure. This is a necessary safety condition so that the tissue temperature is no overheated in order to avoid tearing and charring of the myocardial tissue.

The second modality 61 delivers energy pulses to the myocardium based on either a fixed or variable duty cycle.

A digital temperature readout is provided. The duty cycle for the power delivery can be set by entering appropriate time duration for ON time and OFF time. During the ON-time, microwave energy is delivered to the myocardium and during the OFF-time the microwave generator is in standby mode. The pre-ablation check sequence is also used before tha-pulsed ablation procedure begins.

The third modality 62 modulates the delivered energy within upper and lower bound of desirable tissue temperature.

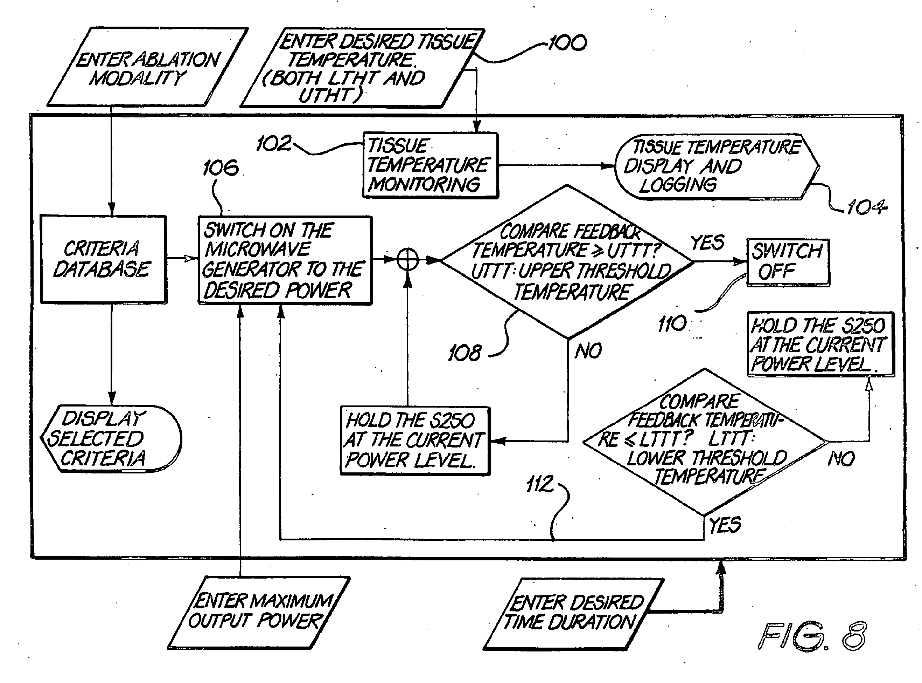

Figure 8 shows the control algorithm. The upper and lower temperatures represent extra control parameters that are required to be entered 100 to achieve this modality. The software also continuously monitors 102 and displays 104 the tissue temperature. During ablation 106 if the tissue temperature is monitored 108 to see whether it has approached or exceeded the upper temperature threshold (UTT). If so, an Instruction to stop 110 the microwave energy delivery is issued to the microwave generator. When the instruction is received, the microwave generator is switched to stand-by mode. When the tissue temperature falls below the lower temperature threshold (LTT) 112, an instruction to begin energy delivery again is sent to the microwave generator. When this instruction is received, the generator begins to deliver microwave energy to the myocardium. This cycle continues until the end of ablation time is reached.

Lastly, the fourth 63 allows the cardiologist to have full manual control. Under this energy delivery mode, the microwave generator can be operated either through the front panel or through the remote control notebook computer. Also, the pre-ablation check is disabled. During ablation, however, the temperature data are still recorded and stored for record keeping and post processing purposes.

### Effect on Lesion Sizes Due to Different Ablation Modalities

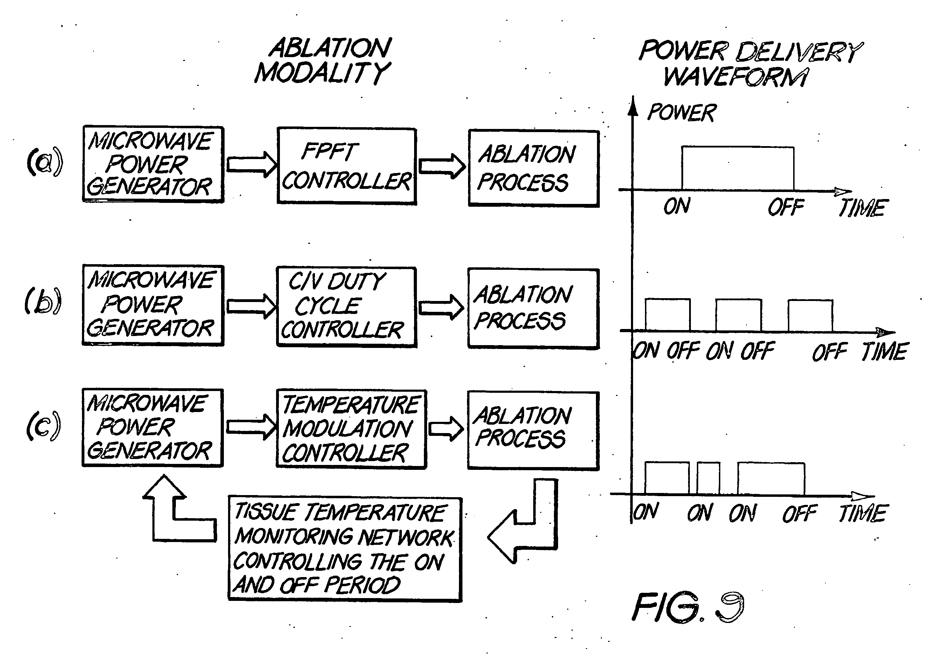

Figure 9 shows the power delivery waveforms associated with ablation modalities. The power delivery waveform for manual mode is not shown because the power being delivered to the myocardium is dependent on the cardiologist operating the ablation system.

Figure 9(a) shows the power waveform for the Fixed-Power Fixed-Time (FPFT) ablation modality. It is clear that for FPFT ablation modality, the power is delivered to the myocardium for a specified time. This is the most common type of ablation modality used because it can be used to deliver high amount of energy in a very short duration to achieve a deep lesion at the cost of wider lesion. This type of ablation modality is useful if large volume of tissue is required to be ablated.

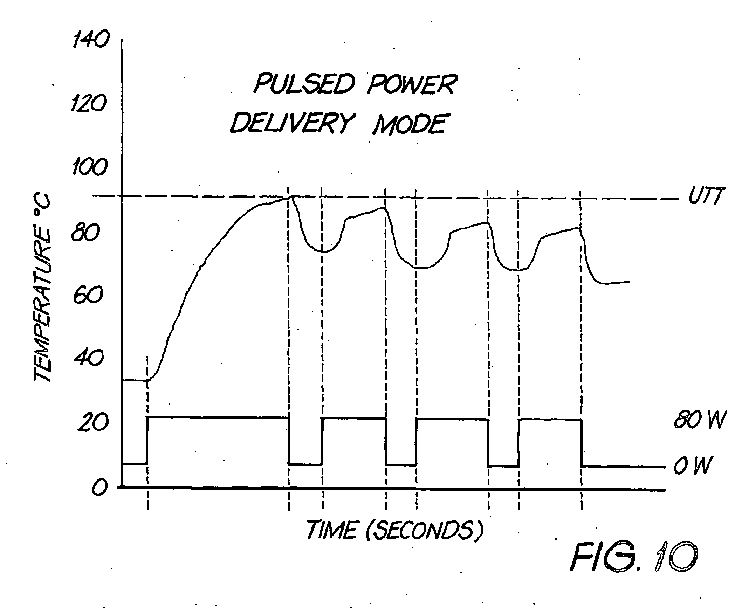

In order to reduce the width of the lesion, areas in the tissue not immediately heated by microwave energy should be allowed to be cooled. One way to achieve this is to introduce a series of OFF periods during energy delivery as shown in Figure 9(b), The temperature and power delivery waveform for the C/V duty cycle pulsed ablation modality. From Figure 10 it can be seen that during the initial power delivery period, the temperature is allowed to rise until it reaches the upper limit of the tissue temperature (UTT) which is preset at 90 °C. Once the preset tissue temperature has been reached, the duty cycle starts and pulsing of microwave energy is initiated. The power waveform shown in Figure 9(b) has a duty cycle of 5:3, that is, the microwave generator is operating at 5 seconds ON and 3 seconds OFF. Note that the preset temperature in this ablation modality can only be changed by physically modifying the program code. From Figures 10, it is clear that the tissue temperature is allowed to be cooled, hence the temperature drop, for the period that the microwave generator is in standby (OFF) mode. This has the effect that the tissues not immediately heated by microwave energy are not heated too much thus reducing the lesion width growth caused by heat conduction. The duty cycle of energy delivery pulse can be made constant, that is 50% ON time and 50% OFF time or variable such as the one shown. Although the pulsed power delivery mode is able to reduce the width of the lesion, this is achieved at the cost of longer ablation duration. This is due to the fact that in order to achieve same lesion depth as the ones achievable using the FPFT ablation modality, longer time is required.

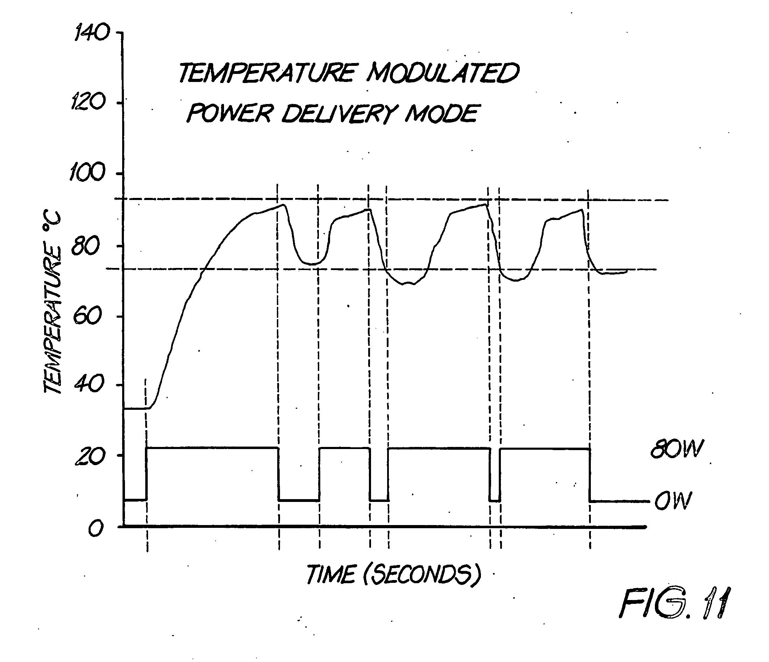

Figure 9(c) shows the power delivery waveform of the Temperature Modulated Pulsed Ablation Modality. Similar to the power delivery waveform shown in Figure 9(b), microwave energy in this modality is delivered by pulsed method. The difference between the C/V duty cycle pulsed modality and the temperature modulated modality is that the duty cycle for the pulse train is not required. Instead, the upper and lower threshold tissue temperature (UTTT and LTTT respectively) are defined. This is shown in Figure 11. During the initial power delivery stage, the temperature is allowed to rise until the UTTT value then the microwave generator enters the standby mode thereby stopping the power delivery. Once the notebook computer detects that the temperature has fallen below the LTTT value the microwave generator is switched back to ON position and the power delivery begins again.

The advantage of using the temperature modulated power delivery modality is that the power being delivered to the myocardial tissue can be made to adapt the state of tissue temperature. For example, due to the high thermal energy produced by the initial microwave irradiation, when the temperature reached the UTTT state the time in which the microwave generator is in OFF status is longer. When the tissue temperature falls near or below the LTTT state, the time in which microwave generator is in ON status is adjusted automatically in order to keep the tissue temperature variation within the UTTT and LTTT state.

If the UTTT is set at 90°C, such as the one shown in Figure 10, then ablation takes affect. If the UTTT is set at around 45 °C, then this system can be utilized in applications such as hyperthermia for cancer treatment. Also if UTTT and LTTT values are made close together, then the microwave generator can be controlled to deliver power which will maintain the temperature within the temperature bounded by UTTT and LTTT. This is not possible with the other types of ablation modality.

Another advantage of this method, over the other method discussed previously, is that the power is delivered dependent on the cooling conditions of the myocardial tissue. If some how the cooling of the myocardial tissue is increased, the microwave generator will be in the ON status much more than in the OFF status. On the other hand if the cooling of the tissue is hindered, then the microwave generator will be in the OFF status longer than in the ON status. When the temperature difference between the UTTT and LTTT is reduced, using the temperature modulated power delivery modality; the tissue temperature can be maintained for a preset time.

**Table 5 shows the lesion sizes obtained using the three power delivery modes. Table 5: Lesion comparisons for the three power delivery modality.**

| Delivery mode/Duration | Depth (mm) | Width (mm) | Surface Area (mm²) | W:D |
|---|---|---|---|---|
| FPFT 80W 30sec | 7.7 | 10.9 | 76.9 | 1.5 |
| C/V Pulse 80/80 | 6.5 | 6.5 | 65 | 1 |
| TM Pulse 80/90 | 6.8 | 5.7 | 73 | 0.84 |

To achieve large lesion size in both depth and width dimensions, the FPFT is the best method. However, as shown in the width to depth (W:D) ratio column, the FPFT also has largest W:D ratio meaning that the lesion generated using the FPFT method is much wider than it is deep. This type of power delivery modality would be very well suited for ablation in the ventricle where large volume of arrhythmogenic tissues are require to be ablated.

On the other hand, the W:D ratio of the C/V duty cycle pulsed (C/V pulse) ablation modality, a width to depth ratio of 1 can be achieved thereby reducing the unnecessary injury to the tissues surrounding the arrhythmogenic tissue. This, however, is achieved at the cost of longer ablation duration.

The temperature modulated pulsed (TM Pulse) ablation modality took the longest time to achieve a 7mm deep lesion. However, it also has the lowest width to depth ratio indicating that the lesion generated using the TM Pulse power delivery modality is deeper than it is wide which is perfectly suited for ablation in the atrium where the tissues surrounding the arrhythmogenic tissue is scarce and should be preserved.

It will be appreciated by persons skilled in the art that numerous variations and/or modifications may be made to the invention as shown in the specific embodiments without departing from the scope of the invention as broadly described. The present embodiments are, therefore, to be considered in all respects as illustrative and not restrictive.

## Claims

1. A microwave antenna (10) for medical ablation, comprising:
a transmission line (11) having an inner conductor (13), an outer conductor (14) and a dielectric insulator (15) to provide insulation between the inner and outer conductor (14); and
an energy-emitting antenna element (12) positioned at the distal end of the transmission line (11) to transmit a microwave near-field, the antenna element (12) having an inner conductor (13) electrically coupled to the inner conductor (13) of the transmission line (11) and a sheath (16) of dielectric insulator (15) around the inner conductor (13); and
a conducting metallic cap (20) that is electrically connected to the distal end of the inner conductor (13), the cap (20) surrounding a length of the sheath (16) of insulator (15), and the dimensions of the cap (20) being determined to provide impedance matching between the antenna element (12) and the transmission line (11), the antenna element (12) being configured with conducting rings (18) spaced apart from each other along the length of the antenna element (12) by slots and **characterized in that** said antenna element is configured by being bent to form an open loop oriented such that the antenna element (12) extends transverse to the longitudinal axis of the transmission line (11).

2. An antenna according to claim 1, wherein the dimensions of the metallic cap (20) that are determined include one or more of:
the length of the cap (20);
the length of the sheath (16) of insulator (15) that is surrounded by the cap (20); and
the radius of the cap (20).

3. An antenna according to claim 1 or 2, wherein the antenna element (12) is built into the end of the transmission line (11), and the cap (20) is fixed to the inner conductor (13) of the transmission line (11).

4. An antenna according to claim 3, wherein a first length of the outer conductor (14) is removed from the distal end of the transmission line (11) to create the antenna element (12).

5. An antenna according to claim 4, wherein a shorter length of the dielectric insulator (15) is removed from the distal end to expose a length of the inner conductor (13) for fixing of the cap (20).

6. An antenna according to claim 5, wherein the dimensions to be determined include:
the length of exposed inner conductor (13) between the distal end of the sheath (16) of insulator (15) and the cap (20).

7. An antenna according to claim 1, wherein the antenna element (12) comprises insulating and conducting rings (19, 18) placed alternately along the length of insulating sheath (16).

8. An antenna according to claim 1, wherein the conducting rings (18) comprise rings of outer conductor (14).

9. An antenna according to claim 1, wherein the cap (20) is made using a conducting ring (18).

10. An antenna according to claim 1, wherein the sizes of the conducting rings (18) and the slots between the conducting rings (18) are selected to determine the shape of the near-field distribution.

11. An antenna according to claim 10, wherein all the conducting rings (18) are the same size, and all the slots between the conducting rings (18) are the same size.

12. An antenna according to claim 11, wherein the conductive rings (18) are twice as wide as the slots between the conducting rings (18).

13. An antenna according to claim 10, wherein the slot and ring sizes gradually increase towards the tip of the antenna to make a forward firing antenna.

14. An antenna according to claim 10, wherein the slot and ring sizes gradually decrease towards the tip of the antenna to make a reverse firing antenna.

15. An antenna according to claim 1 or 2, wherein the dielectric loading produced by the size of the insulator surrounded by the cap (20) is determined to ensure the near field flow terminates at the tip of the antenna rather than at the transmission line/antenna element junction.

16. An antenna according to claim 1 or 2, wherein the antenna comprises a Teflon sheath surrounding at least the antenna element (12).

17. An antenna according to claim 1 or 2, wherein the antenna element (12) is delivered to an ablation site by feeding the transmission line (11) through a catheter.

18. An antenna according to claim 1 or 2, comprising a temperature sensor to sense the temperature of the tissue being ablated by the antenna.

19. An antenna according to claim 18, comprising a microwave generator that delivers energy at 2.45 GHz.

20. An antenna according to claim 19, comprising a computer control system to monitor the ablation process and control the microwave generator.

21. A method for making a microwave antenna (10) for medical ablation, comprising an energy emitting antenna element (12) having an inner conductor (13) and a surrounding sheath (16) of insulation, in use, located at the end of a transmission line (11), the method comprising the steps of:
forming a conductive metallic cap (20) at the distal end of the antenna element (12) such that the cap (20) surrounds a length of the sheath (16) of insulator (15);
electrically coupling the conducting cap (20) to the inner conductor (13) of the antenna element (12);
determining the dimensions of the cap (20) to provide impedance matching between the antenna element (12) and the transmission line (11);
configuring the antenna element (12) with conducting rings (18) spaced apart from each other along the length of the antenna element (12) by slots; and
**characterised by** the step of:
configuring the antenna element (12) by being bent to form an open loop oriented such that the antenna element (12) extends transverse to the longitudinal axis of the transmission line (11).

22. A method according to claim 21, wherein the step of determining the dimensions of the cap (20) includes the steps of:
determining the length of the cap (20):
determining the length of the sheath (16) of insulator (15) that is surrounded by the cap (20); and
determining the radius of the cap (20).

23. A method according to claim 21 or 22, wherein the antenna element (12) is built into the end of the transmission line (11), and the cap (20) is fixed to an inner conductor (13) of the transmission line (11).

24. A method according to claim 23, wherein a first length of the outer conductor (14) of the transmission line (11) is removed from the distal end of the transmission line (11) to create the antenna element (12).

25. A method according to claim 24, wherein a shorter length of the dielectric insulator (15) of the transmission line (11) is removed from the distal end to expose a length of the inner conductor (13) for fixing of the cap (20).

26. A method according to claim 25, comprising the step of:
determining the length of exposed inner conductor (13) between the distal end of the sheath (16) of insulator (15) and the cap (20) before the step of determining the dimensions of the cap (20).

27. A method according to claim 21, comprising the step of spacing the conducting rings (18) apart from each other by insulating rings (19).

28. A method according to claim 27, comprising the following steps before the step of determining the dimensions of the cap (20):
determining the width(s) of the conducting rings (18);
determining the width (s) of the slots; and
determining the length of the antenna element (12) between the end of the outer conductor (14) and the cap (20).

29. A method according to claim 21, 27 or 28, wherein the conducting rings (18) comprise rings of the outer conductor (14) of the transmission line (11).

30. A method according to claims 21, 27 or 28, wherein the cap (20) is made using a conducting ring (18).

31. A method according to any one of claims 21 and 27, comprising the step of selecting the sizes of the conducting rings and the slots between the conducting rings to determine the shape of the near-field distribution.

32. A method according to claim 31, wherein all the conducing rings (18) are the same size, and all the slots between the conducting rings (18) are the same size.

33. A method according to claim 32, wherein the conductive rings (18) are twice as wide as the slots between the conductive rings (18).

34. A method according to claim 31, wherein the slot and ring sizes gradually increase towards the tip of the antenna to make a forward firing antenna.

35. A method according to claim 31, wherein the slot and ring sizes gradually decrease towards the tip of the antenna to make a reverse firing antenna.

36. A method according to claim 21, comprising the step of determining the dielectric loading produced by the size of the insulator surrounded by the cap (20) to ensure the near field flow ends at the tip of the antenna rather than at the transmission line/antenna element junction.

37. A method according to claim 36, comprising the step of determining one or more of the following dimensions before the step of determining the dimensions of the cap (20):
determining the straight length of the sheath (16) of insulator (15) before bending begins;
determining the radius of bending between the transmission line (11) and the open loop;
determining the perpendicular distance between the open loop and the beginning of bending;
determining the radius of the open loop;
determining the length of the cap (20) not surrounding the sheath (16) of insulator (15); and
determining the perpendicular distance between the top of the cap (20) and the transmission line (11).

38. A method according to claim 21, comprising the step of encapsulating the antenna element (12) inside a Teflon sheath.

## Patentansprüche

1. Mikrowellenantenne (10) für medizinische Ablation, umfassend:
eine Transmissionsleitung (11), welche einen inneren Leiter (13), einen äußeren Leiter (14) und einen dielektrischen Isolator (15), um Isolierung zwischen dem inneren und äußeren Leiter (14) bereitzustellen, aufweist; und
ein Energie-emittierendes Antennenelement (12), welches an dem distalen Ende der Transmissionsleitung (11) positioniert ist, um ein Mikrowellen-Nahfeld zu übertragen, wobei das Antennenelement (12) einen inneren Leiter (13), welcher an den inneren Leiter (13) der Transmissionsleitung (11) elektrisch gekoppelt ist, und eine Ummantelung (16) aus dielektrischem Isolator (15) um den inneren Leiter (13) herum aufweist; und
eine leitende metallische Kappe (20), die mit dem distalen Ende des inneren Leiters (13) elektrisch verbunden ist, wobei die Kappe (20) eine Länge der Ummantelung (16) aus Isolator (15) umgibt und die Abmessungen der Kappe (20) bestimmt sind, um eine Impedanzanpassung zwischen den Antennenelement (12) und der Transmissionsleitung (11) bereitzustellen, wobei das Antennenelement (12) mit entlang der Länge des Antennenelements (12) durch Schlitze beabstandeten leitenden Ringen (18) gebildet ist, und **dadurch gekennzeichnet, dass** das Antennenelement durch Biegen gebildet ist, um eine offene Schleife auszubilden, welche so orientiert ist, dass sich das Antennenelement (12) quer zu der Längsachse der Transmissionsleitung (11) erstreckt.

2. Antenne nach Anspruch 1, wobei die Abmessungen der metallischen Kappe (20), die bestimmt sind, eine oder mehrere der folgenden enthalten:
die Länge der Kappe (20);
die Länge der Ummantelung (16) aus Isolator (15), welche von der Kappe (20) umgeben ist; und
den Radius der Kappe (20).

3. Antenne nach Anspruch 1 oder 2, wobei das Antennenelement (12) in das Ende der Transmissionsfeitung (11) eingebaut ist und die Kappe (20) an dem inneren Leiter (13) der Transmissionsleitung (11) fixiert ist.

4. Antenne nach Anspruch 3, wobei eine erste Länge des äußeren Leiters (14) von dem distalen Ende der Transmissionsleitung (11) entfernt ist, um das Antennenelement (12) zu schaffen.

5. Antenne nach Anspruch 4, wobei eine kürzere Länge des dielektrischen Isolators (15) von dem distalen Ende entfernt ist, um eine Länge des inneren Leiters (13) zum Fixieren der Kappe (20) freizulegen.

6. Antenne nach Anspruch 5, wobei die zu bestimmenden Abmessungen enthalten:
die Länge von freigelegtem inneren Leiter (13) zwischen dem distalen Ende der Ummantelung (16) aus Isolator (15) und der Kappe (20).

7. Antenne nach Anspruch 1, wobei das Antennenelement (12) isolierende und leitende Ringe (19, 18) umfasst, welche abwechselnd entlang der Länge der isolierenden Ummantelung (16) platziert sind.

8. Antenne nach Anspruch 1, wobei die leitenden Ringe (18) Ringe aus äußerem Leiter (14) umfassen.

9. Antenne nach Anspruch 1, wobei die Kappe (20) unter Verwendung eines leitenden Rings (18) hergestellt ist.

10. Antenne nach Anspruch 1, wobei die Größen der leitenden Ringe (18) und der Schlitze zwischen den leitenden Ringen (18) gewählt sind, um die Form der Nahfeldverteilung zu bestimmen.

11. Antenne nach Anspruch 10, wobei alle leitenden Ringe (18) die gleiche Größe aufweisen und alle Schlitze zwischen den leitenden Ringen (18) die gleiche Größe aufweisen.

12. Antenne nach Anspruch 11, wobei die leitenden Ringe (18) zweimal so breit sind wie die Schlitze zwischen den leitenden Ringen (18).

13. Antenne nach Anspruch 10, wobei die Schlitz- und Ringgrößen graduell in Richtung der Spitze der Antenne zunehmen, um eine vorwärts strahlende Antenne herzustellen.

14. Antenne nach Anspruch 10, wobei die Schlitz- und Ringgrößen graduell in Richtung der Spitze der Antenne abnehmen, um eine rückwärts strahlende Antenne herzustellen.

15. Antenne nach Anspruch 1 oder 2, wobei die dielektrische Ladung, die durch die Größe des von der Kappe (20) umgebenen Isolators produziert wird, bestimmt ist, um sicherzustellen, dass der Nahfeldfluss an der Spitze der Antenne endet und nicht an dem Transmissionsleitung/Antennenelement-Übergang.

16. Antenne nach Anspruch 1 oder 2, wobei die Antenne eine Teflonummantelung umfasst, welche zumindest das Antennenelement (12) umgibt.

17. Antenne nach Anspruch 1 oder 2, wobei das Antennenelement (12) durch Zuführen der Transmissionsleitung (11) durch einen Katheter an eine Ablationsstelle gebracht wird.

18. Antenne nach Anspruch 1 oder 2, umfassend einen Temperatursensor, um die Temperatur des Gewebes zu erfassen, welches mittels der Antenne abgetragen wird.

19. Antenne nach Anspruch 18, umfassend einen Mikrowellengenerator, welcher Energie bei 2.45 GHz liefert.

20. Antenne nach Anspruch 19, umfassend ein Computersteuersystem zum Überwachen des Ablationsprozesses und Steuern des Mikrowellengenerators.

21. Verfahren zum Herstellen einer Mikrowellenantenne (10) für medizinische Ablation, umfassend ein Energie-emittierendes Antennenelement (12), welches einen inneren Leiter (13) und eine umgebende Ummantelung (16) aus Isolierung aufweist, welches im Gebrauch am Ende einer Transmissionsleitung (11) angeordnet ist, wobei das Verfahren die Schritte umfasst:
Ausbilden einer leitenden metallischen Kappe (20) an dem distalen Ende des Antennenelements (12), so dass die Kappe (20) eine Länge der Ummantelung (16) aus Isolator (15) umgibt;
elektrisches Koppeln der leitenden Kappe (20) an den inneren Leiter (13) des Antennenelements (12);
Bestimmen der Abmessungen der Kappe (20), um eine Impedanzanpassung zwischen dem Antennenelement (12) und der Transmissionsleitung (11) bereitzustellen;
Bilden des Antennenelements (12) mit entlang der Länge des Antennenelements (12) durch Schlitze voneinander beabstandeten leitenden Ringen (18); und
**gekennzeichnet durch** den Schritt:
Bilden des Antennenelements (12) **durch** Biegen, um eine offene Schleife auszubilden, welche so orientiert ist, dass sich das Antennenelement (12) quer zu der Längsachse der Transmissionsleitung (11) erstreckt.

22. Verfahren nach Anspruch 21, wobei der Schritt des Bestimmens der Abmessungen der Kappe (20) die Schritte enthält:
Bestimmen der Länge der Kappe (20);
Bestimmen der Länge der Abschirmung (16) aus Isolator (15), welche von der Kappe (20) umgeben ist; und
Bestimmen des Radius der Kappe (20).

23. Verfahren nach Anspruch 21 oder 22, wobei das Antennenelement (12) in das Ende der Transmissionsleitung (11) eingebaut wird und die Kappe (20) an dem inneren Leiter (13) der Transmissionsleitung (11) fixiert wird.

24. Verfahren nach Anspruch 23, wobei eine erste Länge des äußeren Leiters (14) der Transmissionsleitung (11) von dem distalen Ende der Transmissionsleitung (11) entfernt wird, um das Antennenelement (12) zu schaffen.

25. Verfahren nach Anspruch 24, wobei eine kürzere Länge des dielektrischen Isolators (15) der Transmissionsleitung (11) von dem distalen Ende entfernt wird, um eine Länge des inneren Leiters (13) zum Fixieren der Kappe (20) freizulegen.

26. Verfahren nach Anspruch 25, umfassend den Schritt:
Bestimmen der Länge des freigelegten inneren Leiters (13) zwischen dem distalen Ende der Ummantelung (16) aus Isolator (15) und der Kappe (20) vor dem Schritt des Bestimmens der Abmessungen der Kappe (20).

27. Verfahren nach Anspruch 21, umfassend den Schritt des Beabstandens der leitenden Ringe (18) voneinander durch isolierende Ringe (19).

28. Verfahren nach Anspruch 27, umfassend die folgenden Schritte vor dem Schritt des Bestimmens der Abmessungen der Kappe (20):
Bestimmen der Breite(n) der leitenden Ringe (18);
Bestimmen der Breite(n) der Schlitze: und
Bestimmen der Länge des Antennenelements (12) zwischen dem Ende des äußeren Leiters (14) und der Kappe (20).

29. Verfahren nach Anspruch 21, 27 oder 28, wobei die leitenden Ringe (18) Ringe aus äußerem Leiter (14) der Transmissionsleitung (11) umfassen.

30. Verfahren nach Anspruch 21, 27 oder 28, wobei die Kappe (20) unter Verwendung eines leitenden Rings (18) hergestellt wird.

31. Verfahren nach einem der Ansprüche 21 und 27, umfassend den Schritt des Auswählens der Größen der leitenden Ringe und der Schlitze zwischen den leitenden Ringen, um die Form der Nahfeldverteilung zu bestimmen.

32. Verfahren nach Anspruch 31, wobei alle leitenden Ringe (18) die gleiche Größe aufweisen und alle Schlitze zwischen den leitenden Ringen (18) die gleiche Größe aufweisen.

33. Verfahren nach Anspruch 32, wobei die leitenden Ringe (18) zweimal so breit sind wie die Schlitze zwischen den leitenden Ringen (18).

34. Verfahren nach Anspruch 31, wobei die Schlitz- und Ringgrößen graduell in Richtung der Spitze der Antenne zunehmen, um eine vorwärts strahlende Antenne herzustellen.

35. Verfahren nach Anspruch 31, wobei die Schlitz- und Ringgrößen graduell in Richtung der Spitze der Antenne abnehmen, um eine rückwärts strahlende Antenne herzustellen.

36. Verfahren nach Anspruch 21, umfassend den Schritt des Bestimmens der dielektrischen Ladung, die durch die Größe des von der Kappe (20) umgebenen Isolators produziert wird, um sicherzustellen, dass der Nahfeldfluss an der Spitze der Antenne endet und nicht an dem Transmissionsleitung/Antennenelement-Übergang.

37. Verfahren nach Anspruch 36, umfassend den Schritt des Bestimmens einer oder mehrerer der folgenden Abmessungen vor dem Schritt des Bestimmens der Abmessungen der Kappe (20):
Bestimmen der geraden Länge der Ummantelung (16) aus Isolator (15) bevor eine Biegung beginnt;
Bestimmen des Biegeradius zwischen der Transmissionsleitung (11) und der offenen Schleife;
Bestimmen des senkrechten Abstands zwischen der offenen Schleife und dem Biegungsbeginn;
Bestimmen des Radius der offenen Schleife;
Bestimmen der Länge der Kappe (20), welche die Ummantelung (16) aus Isolator (15) nicht umgibt; und
Bestimmen des senkrechten Abstands zwischen dem oberen Ende der Kappe (20) und der Transmissionsleitung (11).

38. Verfahren nach Anspruch 21, umfassend den Schritt des Einkapselns des Antennenelements (12) in einer Teflonummantelung.

## Revendications

1. Antenne à hyperfréquences (10) pour une ablation médicale, comprenant :
une ligne de transmission (11) ayant un conducteur interne (13), un conducteur externe (14) et un isolateur diélectrique (15) pour fournir une isolation entre les conducteurs interne et externe (14) ; et
un élément d'antenne d'émission d'énergie (12) positionné au niveau de l'extrémité distale de la ligne de transmission (11) afin de transmettre un champ proche d'hyperfréquence, l'élément d'antenne (12) ayant un conducteur interne (13) couplé de manière électrique au conducteur interne (13) de la ligne de transmission (11) et une gaine (16) d'isolateur diélectrique (15) autour du conducteur interne (13) ; et
un capuchon métallique conducteur (20) qui est raccordé électriquement à l'extrémité distale du conducteur interne (13), le capuchon (20) entourant une longueur de la gaine (16) de l'isolateur (15), et les dimensions du capuchon (20) étant déterminées pour fournir la correspondance d'impédance entre l'élément d'antenne (12) et la ligne de transmission (11), l'élément d'antenne (12) étant configuré avec des bagues conductrices (18) espacées les unes des autres le long de la longueur de l'élément d'antenne (12), par des fentes et **caractérisée en ce que** ledit élément d'antenne est configuré en étant plié pour former une boucle ouverte orientée de sorte que l'élément d'antenne (12) s'étend de manière transversale par rapport à l'axe longitudinal de la ligne de transmission (11).

2. Antenne selon la revendication 1, dans laquelle les dimensions d'un capuchon métallique (20) qui sont déterminées, comprennent un ou plusieurs parmi :
la longueur du capuchon (20) ;
la longueur de la gaine (16) de l'isolateur (15) qui est entouré par le capuchon (20) ; et
le rayon du capuchon (20).

3. Antenne selon la revendication 1 ou 2, dans laquelle l'élément d'antenne (12) est construit dans l'extrémité de la ligne de transmission (11), et le capuchon (20) est fixé sur le conducteur interne (13) de la ligne de transmission (11).

4. Antenne selon la revendication 3, dans laquelle une première longueur du conducteur externe (14) est retirée de l'extrémité distale de la ligne de transmission (11) afin de créer l'élément d'antenne (12).

5. Antenne selon la revendication 4, dans laquelle une longueur plus courte de l'isolateur diélectrique (15) est retirée de l'extrémité distale afin d'exposer une longueur du conducteur interne (13) pour la fixation du capuchon (20).

6. Antenne selon la revendication 5, dans laquelle les dimensions à déterminer comprennent :
la longueur du conducteur interne (13) exposé entre l'extrémité distale de la gaine (16) de l'isolateur (15) et le capuchon (20).

7. Antenne selon la revendication 1, dans laquelle l'élément d'antenne (12) comprend des bagues d'isolation et conductrices (19, 18) placées de manière alternée le long de la longueur de la gaine isolante (16).

8. Antenne selon la revendication 1, dans laquelle les bagues conductrices (18) comprennent des bagues de conducteur externe (14).

9. Antenne selon la revendication 1, dans laquelle le capuchon (20) est réalisé en utilisant une bague conductrice (18).

10. Antenne selon la revendication 1, dans laquelle les tailles des bagues conductrices (18) et les fentes entre les bagues conductrices (18) sont sélectionnées pour déterminer la forme de la distribution de champ proche.

11. Antenne selon la revendication 10, dans laquelle toutes les bagues conductrices (18) sont de la même taille, et toutes les fentes entre les bagues conductrices (18) sont de la même taille.

12. Antenne selon la revendication 11, dans laquelle les bagues conductrices (18) sont deux fois plus larges que les fentes entre les bagues conductrices (18).

13. Antenne selon la revendication 10, dans laquelle les tailles de la fente et de la bague augmentent progressivement vers la pointe de l'antenne afin de réaliser une antenne de cautérisation vers l'avant.

14. Antenne selon la revendication 10, dans laquelle les tailles de la fente et de la bague diminuent progressivement vers la pointe de l'antenne, afin de réaliser une antenne de cautérisation vers l'arrière.

15. Antenne selon la revendication 1 ou 2, dans laquelle la charge diélectrique produite par la taille de l'isolateur entouré par le capuchon (20) est déterminée pour garantir l'écoulement de champ proche qui se termine au niveau de la pointe de l'antenne plutôt qu'au niveau de la jonction de la ligne de transmission / élément d'antenne.

16. Antenne selon la revendication 1 ou 2, dans laquelle l'antenne comprend une gaine en Teflon entourant au moins l'élément d'antenne (12).

17. Antenne selon la revendication 1 ou 2, dans laquelle l'élément d'antenne (12) est délivré sur un site d'ablation en alimentant la ligne de transmission (11) par le biais d'un cathéter.

18. Antenne selon la revendication 1 ou 2, comprenant un capteur de température pour détecter la température du tissu qui est ablaté par l'antenne.

19. Antenne selon la revendication 18, comprenant un générateur d'hyperfréquences qui délivre de l'énergie à 2,45 GHz.

20. Antenne selon la revendication 19, comprenant un système de commande par ordinateur pour surveiller l'ablation et contrôler le générateur d'hyperfréquences.

21. Procédé pour fabriquer une antenne à hyperfréquences (10) pour une ablation médicale, comprenant un élément d'antenne d'émission d'énergie (12) ayant un conducteur interne (13) et une gaine (16) périphérique d'isolation, à l'usage, positionnée au niveau de l'extrémité d'une ligne de transmission (11), le procédé comprenant les étapes consistant à :
former un capuchon métallique conducteur (20) au niveau de l'extrémité distale de l'élément d'antenne (12) de sorte que le capuchon (20) entoure une longueur de la gaine (16) de l'isolateur (15) ;
coupler électriquement le capuchon conducteur (20) au conducteur interne (13) de l'élément d'antenne (12) ;
déterminer les dimensions du capuchon (20) pour fournir l'impédance correspondant entre l'élément d'antenne (12) et la ligne de transmission (11) ;
configurer l'élément d'antenne (12) avec des bagues conductrices (18) espacées les unes des autres le long de la longueur de l'élément d'antenne (12) par des fentes ; et
**caractérisé par** l'étape consistant à :
configurer l'élément d'antenne (12) en étant plié afin de former une boucle ouverte orientée de sorte que l'élément d'antenne (12) s'étend de manière transversale par rapport à l'axe longitudinal de la ligne de transmission (11).

22. Procédé selon la revendication 21, dans lequel l'étape consistant à déterminer les dimensions du capuchon (20) comprend les étapes consistant à :
déterminer la longueur du capuchon (20) ;
déterminer la longueur de la gaine (16) de l'isolateur (15) qui est entourée par le capuchon (20) ; et
déterminer le rayon du capuchon (20) ;

23. Procédé selon la revendication 21 ou 22, dans lequel l'élément d'antenne (12) est construit dans l'extrémité de la ligne de transmission (11), et le capuchon (20) est fixé sur un conducteur interne (13) de la ligne de transmission (11).

24. Procédé selon la revendication 23, dans lequel une première longueur du conducteur externe (14) de la ligne de transmission (11) est retirée de l'extrémité distale de la ligne de transmission (11) afin de créer l'élément d'antenne (12).

25. Procédé selon la revendication 24, dans lequel une longueur plus courte de l'isolateur diélectrique (15) de la ligne de transmission (11) est retirée de l'extrémité distale afin d'exposer une longueur du conducteur interne (13) pour la fixation du capuchon (20).

26. Procédé selon la revendication 25, comprenant l'étape consistant à :
déterminer la longueur du conducteur interne (13) exposé entre l'extrémité distale de la gaine (16) de l'isolateur (15) et le capuchon (20) avant l'étape consistant à déterminer les dimensions du capuchon (20).

27. Procédé selon la revendication 21, comprenant l'étape consistant à espacer les bagues conductrices (18) les unes des autres par des bagues isolantes (19).

28. Procédé selon la revendication 27, comprenant les étapes suivantes avant l'étape consistant à déterminer les dimensions du capuchon (20), consistant à :
déterminer la (les) largeur(s) des bagues conductrices (18) ;
déterminer la (les) largeur(s) des fentes ; et
déterminer la longueur de l'élément d'antenne (12) entre l'extrémité du conducteur externe (14) et le capuchon (20).

29. Procédé selon la revendication 21, 27 ou 28, dans lequel les bagues conductrices (18) comprennent des bagues du conducteur externe (14) de la ligne de transmission (11).

30. Procédé selon les revendications 21, 27 ou 28, dans lequel le capuchon (20) est réalisé en utilisant une bague conductrice (18).

31. Procédé selon l'une quelconque des revendications 21 et 27, comprenant l'étape consistant à sélectionner les tailles des bagues conductrices et des fentes entre les bagues conductrices afin de déterminer la forme de la distribution de champ proche.

32. Procédé selon la revendication 31, dans lequel toutes les bagues conductrices (18) sont de la même taille et toutes les fentes entre les bagues conductrices (18) sont de la même taille.

33. Procédé selon la revendication 32, dans lequel les bagues conductrices (18) sont deux fois plus larges que les fentes entre les bagues conductrices (18).

34. Procédé selon la revendication 31, dans lequel les tailles de la fente et de la bague augmentent progressivement vers la pointe de l'antenne afin de réaliser une antenne de cautérisation vers l'avant.

35. Procédé selon la revendication 31, dans lequel les tailles de la fente et de la bague diminuent progressivement vers la pointe de l'antenne afin de réaliser une antenne de cautérisation vers l'arrière.

36. Procédé selon la revendication 21, comprenant l'étape consistant à déterminer la charge diélectrique produite par la taille de l'isolateur entouré par le capuchon (20) pour garantir que l'écoulement de champ proche se termine au niveau de la pointe de l'antenne plutôt qu'au niveau de la jonction de ligne de transmission / élément d'antenne.

37. Procédé selon la revendication 36, comprenant l'étape consistant à déterminer une ou plusieurs des dimensions suivantes avant l'étape consistant à déterminer les dimensions du capuchon (20) :
déterminer la longueur droite de la gaine (16) de l'isolateur (15) avant que le cintrage ne commence ;
déterminer le rayon de courbure entre la ligne de transmission (11) et la boucle ouverte ;
déterminer la distance perpendiculaire entre la boucle ouverte et le début du cintrage ;
déterminer le rayon de la boucle ouverte ;
déterminer la longueur du capuchon (20) n'entourant pas la gaine (16) de l'isolateur (15) ; et
déterminer la distance perpendiculaire entre la partie supérieure du capuchon (20) et la ligne de transmission (11).

38. Procédé selon la revendication 21, comprenant l'étape consistant à encapsuler l'élément d'antenne (12) à l'intérieur d'une gaine en Téflon.
